# EUROPÄISCHE PATENTANMELDUNG

(11) **EP 1 975 246 A1**
(43) Veröffentlichungstag der Anmeldung: **01.10.2008**
(21) Anmeldenummer: 07006563.6
(22) Anmeldetag: 29.03.2007
(51) Int. Cl.: C12Q 1/68

(54) **Markierungsfreie Sequenzierung auf einer Festphase mittels Feldeffekttransistoren**

(71) Anmelder: Micronas Holding GmbH, 79108 Freiburg (DE)
(72) Erfinder: Klapproth, Holger, 79108 Freiburg (DE); Lehmann, Mirko, 79117 Freiburg (DE)
(74) Vertreter: Stürken, Joachim

(57) **Zusammenfassung**

Die vorliegende Erfindung betrifft ein Verfahren zur markierungsfreien Sequenzierung von Nukleinsäuren, bei dem lediglich eine Art von Enzym eingesetzt wird.

Das erfindungsgemäße Verfahren umfasst die folgenden Schritte:
a) Inkubation von auf einer festen Phase immobilisierten DNA-Sonden mit einzelsträngigen Nukleinsäureanalyten zur Ausbildung eines Hybridisierungskomplexes;
b) Zugabe eines der vier Nukleosidtriphosphate dATP, dCTP, dGTP und dTTP;
c) Einbau der Nukleosidtriphospate in Anwesenheit der DNA-Polymerase oder des Fragments derselben, wodurch Pyrophosphatanionen freigesetzt und von einem Sensor detektiert werden;
d) Entfernung überschüssiger Nukleosidtriphosphate;
e) wiederholung der Schritte b) bis d) unter Zugabe jeweils anderer Nukleosidtriphosphate; sowie optional
f) Mehrfache Wiederholung der Schritte b) bis e); und
g) Bestimmung der Sequenz des Nukleinsäureanalyten auf der Basis der Messdaten, die im jeweiligen Schritt c) erfasst worden sind,

wobei die Detektion in Schritt c) durch mindestens einen Sensor erfolgt, der monolithisch in der festen Phase integriert ist.

## Beschreibung

Die vorliegende Erfindung betrifft ein Verfahren zur markierungsfreien Sequenzierung einer oder mehrerer Nukleinsäuren, bei dem lediglich eine Art von Enzym eingesetzt wird.

Eine Sequenzierung wird vorgenommen, um die Nukleotidabfolge innerhalb eines Nukleinsäuremoleküls zu bestimmen. Aus dem Stand der Technik sind zahlreiche Verfahren zur Sequenzierung von Nukleinsäuren bekannt.

Die Didesoxymethode nach Sanger stellt eine enzymatische Methode dar. Bei dieser Methode wird ausgehend von einem kurzen Abschnitt bekannter Sequenz (Primer) einer der beiden komplementären DNA-Stränge mit Hilfe der DNA-Polymerase verlängert. Hierfür werden Didesoxynukleosidtriphospate (ddNTP) zugegeben. Sofern die ddNTPs in den neusynthetisierten Strang eingebaut werden, ist eine weitere DNA-Verlängerung durch eine DNA-Polymerase nicht mehr möglich, da die OH-Gruppe am 3'-C-Atom für die Verknüpfung mit der Phosphatgruppe des nächsten Nukleotids fehlt. In der Folge entstehen DNA-Fragmente unterschiedlicher Länge, wobei entweder der Primer oder das ddNTP markiert ist. Seit Anfang der neunziger Jahre werden vor allem mit Fluoreszenz-Farbstoffen markierte Didesoxynukleosidtriphosphate eingesetzt. Hierbei wird jedes der vier ddNTP mit einem unterschiedlichen Farbstoff gekoppelt. Diese Modifikation ermöglicht die gleichzeitige Zugabe aller vier ddNTP in ein Reaktionsgefäß, wodurch eine Aufspaltung in getrennte Ansätze sowie der Umgang mit Radioisotopen entfallen. Die entstehenden Kettenabbruchprodukte werden mittels Kapillar-Gelelektrophorese aufgetrennt und mit Hilfe eines Lasers zur Fluoreszenz angeregt. Die ddNTPs eines jeden DNA-Fragmentes zeigen dadurch Fluoreszenz unterschiedlicher Farbe und können so von einem Detektor erkannt werden.

Aus der Abfolge der Farbsignale, die vom Detektor erkannt werden, ergibt sich direkt die Sequenz der Basen des sequenzierten DNA-Stranges.

Eine weitere Methode zur DNA-Sequenzierung ist die sogenannte "Pyrosequenzierung", die in erster Linie zur Sequenzierung kurzer DNA-Abschnitte sowie zur Bestimmung der Häufigkeit von bestimmten Genmutationen (SNPs, engl. single nucleotide polymorphism) eingesetzt wird. Bei dieser Methode wird der erfolgreiche Einbau eines Nukleotids mit Hilfe eines Enzymsystems unter Beteiligung der DNA-Polymerase, ATP-Sulfurylase, Luziferase und Apyrase in ein Lichtsignal umgewandelt, das von einem Detektor erfasst wird. Die zu sequenzierende DNA liegt einzelsträngig vor und dient als Matrizenstrang. Ausgehend von einem Primer erfolgt die Strangverlängerung durch kontrollierte Zugabe von dNTP, wobei für jeden Schritt der Strangverlängerung jeweils nur ein Nukleotidtyp, also entweder A, G, C oder T, bereitgestellt wird. Bei dem Einbau von komplementären Nukleosiden wird entsprechend der Menge an eingebauten dNTP Pyrophosphat (PPi) freigesetzt. Die ATP-Sulfurylase setzt das PPi in Anwesenheit von Adenosin-5'Phosphosulfat quantitativ zu ATP um. Das ATP steuert wiederum die Umsetzung von Luziferin zu Oxyluziferin, was durch die Luziferase vermittelt wird. Die dabei generierte Lichtmenge wird von einer CCD-Kamera detektiert, wobei jedes Lichtsignal proportional zur Anzahl der eingebauten Nukleotide ist. Bei nicht-komplementären dNTPs wird entsprechend kein Lichtsignal generiert. Nach jedem einzelnen Schritt der Strangverlängerung werden zunächst überschüssige dNTPs durch das Nukleotide abbauende Enzym Apyrase aus der Lösung entfernt, bevor ein folgender Schritt der Strangverlängerung durchgeführt werden kann. Dies ist erforderlich, damit es zu keiner Signalvermischung kommt.

Die Technologie der Pyrosequenzierung ist von der Firma 454 Life Sciences weiterentwickelt worden und ist unter dem Begriff "454-Seqenzierung" bekannt. Die Methode beinhaltet die Genomsequenzierung unter Verwendung eines bestimmten Geräts, welches innerhalb von vier Stunden die Verarbeitung von ca. 20 Millionen Nukleotidbasen ermöglicht. Die Technologie basiert auf der Kombination von lichtemittierenden chemischen Sequenzierungsmethoden und Informatikverfahren. DNA-Fragmente werden auf Beads fixiert und mittels PCR amplifiziert. Diese DNA-Beads werden dann einem Chip (sogennante PicoTiterPlate) zugeführt, und es wird ein Mix aus verschiedenen Enzymen wie DNA-Polymerase, Sulfurylase und Luziferase hinzugegeben. Anschließend wird die PicoTiterPlate in das Gerät GS20 plaziert, wobei die vier Nukleotide (T, A, G, C) nacheinander über die PicoTiterPlate gespült werden. Während des Nukleotid-Durchflusses werden Hunderttausende der Beads mit Millionen DNA-Kopien parallel sequenziert. Sofern ein zum Matrizenstrang komplementäres Nukleotid in eine Vertiefung der PicoTiterPlate gelangt, verlängert die DNA-Polymerase den bestehenden DNA-Strang durch den Einbau eines oder ggf. mehrerer Nukleotide, was zu einer lichtemittierenden Reaktion führt, wobei das generierte Lichtsignal im GS20-Gerät von einer CCD-Kamera aufgezeichnet wird. Die Signalstärke ist hierbei proportional zur Anzahl der eingebauten Nukleotide. Unter Verwendung von Bildverarbeitungsprogrammen und spezifischer Datenanalyse werden die erhaltenen Resultate schließlich ausgewertet.

Nachteilig bei der 454-Seqenzierung und der Pyrosequenzierung ist, dass die jeweils abgelesenen Abschnitte mit maximal etwa 100 Basenpaaren vergleichsweise kurz sind, was den Sequenzierungsprozess ganzer Genome erschwert. Insbesondere bei der Sequenzierung hochrepetitiver Genome können Probleme auftreten, weil repetitive Bereiche mit mehr als 100 Basen nicht überbrückt werden können. Darüber hinaus kann die Sequenzierung langer Homopolymere problematisch sein.

Beispielsweise kann ein Abschnitt mit 7 A nicht mit hoher Sicherheit von einem Abschnitt mit 8 A unterschieden werden.

Die EP 1456417 A2 beschreibt ein Verfahren zum Nachweis des Vorhandenseins eines Nukleinsäureanalyten in einer zu untersuchenden Probe ohne die herkömmliche Nutzung von optisch nachweisbaren Markersubstanzen. An einer festen Phase sind geeignete DNA-Sonden bzw. Primer immobilisiert. Im Falle des Vorhandenseins eines hierzu komplementären Analyten in der zu untersuchenden Probe kommt es zur Ausbildung eines Hybridisierungskomplexes. Anschließend wird der Komplex mit einem geeigneten Enzym inkubiert, welches zum Aufbau oder Abbau des teilweise doppelsträngigen Komplexes und damit zu einer Massenveränderung desselben führt, wobei jedoch nicht die Veränderung des Gewichts des Komplexes, sondern die durch die Enzymleistung bedingten elektrischen und/oder chemischen Veränderungen in unmittelbarer Umgebung des Komplexes der Detektion dienen. Die Detektion selbst erfolgt durch Sensoren, die integraler Bestandteil der festen Phase sind. Die Messung des Auf- oder Abbaus des Hybridisierungskomplexes erfolgt innerhalb eines Zeitfensters von wenigen Sekunden oder Minuten, wodurch die üblicherweise anzutreffende Drift-Problematik umgangen wird. Generiert wird ein analoges Signal, das zu jedem Zeitpunkt des Aufbaus des Hybridisierungskomplexes gemessen wird.

Die EP 1046421 B1 beschreibt eine Polymersynthese unter Verwendung eines Monomers, das eine durch einen Aktivator entfernbare Schutzgruppe aufweist. Es werden ferner Verbindungen mit photoentfernbaren Schutzgruppen sowie ein Reaktorsystem zum Synthetisieren von Polymersequenzen und ein Verfahren zum Screening von Linkerpolymeren zur Verwendung in Bindungsstudien beschrieben. Eine Schutzgruppe wird an eine Monomereinheit chemisch gebunden und kann durch die selektive Exposition gegenüber einem Aktivator entfernt werden.

Als Schutzgruppen werden die folgenden Verbindungen Nitropirenoyl, Pyrenylmethoxycarbonyl, Nitroveratryl, Nitrobenzyl, Dimethyldimethoxybenzyl, 5-Brom-7-nitroindolinyl, o-Hydroxy-α-methylcinnamoyl und 2-Oxymethylenanthrachinon angeführt.

Sakata et al. ("Direct transduction of allele-specific primer extension into electrical signal using genetic field effect transistor", Biosensors and Bioelectronics, BIOS-2082, www.sciencedirect.com, 2006) beschreiben eine SNP-Genotypisierung mit Hilfe eines Feldeffekttransistors (FET) auf der Basis einer potentiometrischen Detektion. Mit dieser Methode konnte zwischen drei untersuchten Genotypen unterschieden werden, ohne dass die DNA-Analyten markiert wurden, wobei die Methode lediglich eine qualitative Aussage im Hinblick auf die Unterschiedlichkeit der verschiedenen Genotypen erlaubt. Eine quantitative Aussage im Sinne einer präzisen Sequenzierung ist mit der von Sakata et al. beschriebenen Methode nicht möglich. Auf der Gate-Oberfläche immobilisierte Oligonukleotidsonden wurden mit DNA-Analyten hybridisiert. Eine Veränderung der Ladungsdichte auf der Gate-Oberfläche eines FET kann während der Primer-Extensionsreaktion bestimmt werden. Die verwendeten Oligonukleotidsonden hatten eine Basenlänge von 11 Basen, während die DNA-Analyten eine Basenlänge zwischen 21 bis 61 Basen aufwiesen. Die DNA-Polymerase wurde zusammen mit allen vier dNTPs zugegeben.

Die oben beschriebenen Verfahren sind insbesondere nachteilig im Hinblick auf den Aufwand, der geleistet werden muss, um ein geeignetes Signal für eine Sequenzierung zu generieren. So ist es sowohl bei der Pyrosequenzierung als auch bei der "454-Sequenzierung" notwendig, dass verschiedene Enzyme wie DNA-Polymerase, ATP-Sulfurylase, Luziferase und Apyrase eingesetzt werden, damit ein geeignetes Signal generiert werden kann.

Aufgrund dieser Tatsache können auch Probleme im Hinblick auf die Reproduzierbarkeit, Standardisierbarkeit sowie die Sensitivität der Verfahren auftreten. Beispielsweise ist es möglich, dass eine Charge eines der verwendeten Enzyme nicht die notwendige biologische Aktivität aufweist, was das resultierende Verfahrensergebnis verfälschen könnte. Allein durch die Verwendung mehrerer Enzyme erhöht sich somit die Wahrscheinlichkeit für ein fehlerhaftes Ergebnis.

Wie bereits oben dargestellt, treten bei den Verfahren des Standes der Technik ferner Probleme bei der Sequenzierung hochrepetitiver Genome auf.

Die Aufgabe der vorliegenden Erfindung besteht daher in der Bereitstellung eines verbesserten Verfahrens, mit dem die beschriebenen Nachteile des Standes der Technik überwunden sowie der Aufwand für die Generierung eines zur Sequenzierung geeigneten Signals verringert werden.

Die Aufgabe wird erfindungsgemäß durch das Verfahren gemäß Hauptanspruch gelöst. Bevorzugte Ausführungsformen des Verfahrens sind in den Unteransprüchen ausgeführt.

Allgemein betrifft die vorliegende Erfindung somit ein Verfahren zur markierungsfreien Sequenzierung eines Nukleinsäure-Einzelstranges, bei dem lediglich eine DNA-Polymerase oder ein funktionelles Fragment derselben eingesetzt wird. Das erfindungsgemäße Verfahren umfasst die folgenden Schritte:
a) Inkubation von auf einer festen Phase immobilisierten DNA-Sonden mit einzelsträngigen Nukleinsäureanalyten zur Ausbildung eines Hybridisierungskomplexes;
b) Zugabe eines der vier Nukleosidtriphosphate dATP, dCTP, dGTP und dTTP;
c) Einbau der Nukleosidtriphospate in Anwesenheit der DNA-Polymerase oder des Fragments derselben, wodurch Pyrophosphatanionen freigesetzt und von einem Sensor detektiert werden;
d) Entfernung überschüssiger Nukleosidtriphosphate;
e) Wiederholung der Schritte b) bis d) unter Zugabe jeweils anderer Nukleosidtriphosphate; sowie optional
f) Mehrfache Wiederholung der Schritte b) bis e); und
g) Bestimmung der Sequenz des Nukleinsäureanalyten auf der Basis der Messdaten, die im jeweiligen Schritt c) erfasst worden sind,
wobei die Detektion in Schritt c) durch mindestens einen Sensor erfolgt, der monolithisch in der festen Phase integriert ist.

Durch diesen Lösungsansatz gelingt es erfindungsgemäß, ein zur Sequenzierung geeignetes Signal mit verringertem Aufwand zu erzeugen.

Die DNA-Polymerase oder das funktionelle Fragment derselben (wie z.B. das Klenow-Fragment) kann entweder jeweils neu zugegeben werden, oder es verbleibt im Ansatz und kann im nächsten Enzymschritt weiter verwendet werden.

Die Zugabe und/oder das Entfernen der Nukleoside können durch herkömmliches Waschen oder vorzugsweise durch Anlegen und Nutzung eines elektrischen Feldes erfolgen. Das Anlegen eines geeigneten elektrischen Feldes bietet insbesondere den Vorteil, dass das Enzym am Ort der Verfahrensschritte verbleibt und damit nicht neu zugeführt werden muss.

Nach einer bevorzugten Ausführungsform wird der die freigesetzten Pyrophosphatanionen detektierende mindestens eine Sensor ausgewählt aus Feldeffekttransistoren, wobei die Auswahl eines ionensensitiven Feldeffekttransistors (ISFET) besonders bevorzugt ist. Ein ISFET weist eine ionensensitive Schicht auf, mit der die Ionenkonzentration des umgebenden Milieus erfasst werden kann. Je nach Ionenzusammensetzung bzw. -konzentration dieser Flüssigkeit werden im leitenden Kanal Ladungsträger verdrängt oder angereichert. Dadurch ändert sich der elektrische Widerstand, der vom Sensor gemessen wird.

Das die Extension des kürzeren Nukleinsäurestranges des Hybridisierungskomplexes bewirkende Enzym ist eine DNA- oder RNA-abhängige DNA-Polymerase, wobei die Verwendung des Klenow-Fragments der DNA-Polymerase I bevorzugt ist. Weitere bevorzugte DNA-Polymerasen sind thermostabile DNA-Polymerasen, RNA-abhängige DNA-Polymerasen (bevorzugt das Fragment ohne RNase H-Aktivität), sowie funktionelle Fragmente derselben. Im Falle der Verwendung einer RNA-abhängigen DNA-Polymerase können erfindungsgemäß auch RNA-Analyten sequenziert werden. Erfindungsgemäß werden durch die DNA-Extension Pyrophosphatanionen freigesetzt, die zu einer Veränderung des elektrischen Widerstandes führen, welche von einem ISFET gemessen wird. Der spezifische Nachweisbereich umfasst eine Mehrzahl von DNA-Sonden oder DNA-Primern derselben Sequenz. Jedem dieser Nachweisbereiche ist mindestens ein Sensor zugeordnet, so dass ein jeder Nachweis sequenzspezifisch am Ort der enzymbedingten Freisetzung von Pyrophosphatanionen erfolgen kann.

Abhängig von der Anzahl der eingebauten Nukleoside wird eine entsprechende Menge an Pyrophosphatanionen freigesetzt, was zu einer entsprechenden Veränderung des elektrischen Widerstandes führt, was wiederum vom einem ISFET quantifizierbar detektiert wird. Somit entsteht bei dem Einbau eines einzelnen Nukleosids auf dem ISFET ein Signal der Größe n.

Sofern zwei Nukleoside eingebaut werden, entsteht auf dem ISFET entsprechend ein Signal der Größe 2n. Werden also X Nukleoside eingebaut, wird auf dem ISFET folglich ein Signal der Größe Xn generiert.

Aufgrund der Tatsache, dass die Freisetzung von Pyrophosphatanionen zu einer lokalen Ansäuerung und damit zu einer Absenkung des pH-Wertes führt, ist es erfindungsgemäß möglich, diese spezifische Änderung des pH-Wertes durch lokale Anordnung eines ISFETs ortsspezifisch zu detektieren.

Eine bevorzugte Ausführungsform des erfindungsgemäßen Verfahrens betrifft die Verwendung von Nukleosidtriphosphaten mit einer abspaltbaren 3'OH Schutzgruppe, deren Abspaltung jeweils vor dem nächsten Verfahrensschritt b) erfolgt. Wie bereits oben erläutert, hybridisieren einzelsträngige Nukleinsäureanalyte mit auf einer festen Phase immobilisierten komplementären DNA-Sonden. Nach dem Einbau eines dNTP mit einer 3'OH Schutzgruppe ist eine DNA-Extension durch eine DNA-Polymerase aufgrund des geschützten 3'-C-Atoms nicht mehr möglich. Somit können in einer einzelnen Verfahrensstufe keine Signale der Größe 2n oder >2n generiert werden. Wie zuvor erläutert, erfolgt anschließend die Entfernung überschüssiger, d.h. nicht eingebauter dNTPs mit Schutzgruppe, bevor die Zugabe des nächsten dNTPs erfolgt.

Nachdem die vier dNTPs sukzessiv zugegeben worden sind, werden die Schutzgruppen anschließend entfernt und es kann ein neuer Zyklus beginnen.

Erfindungsgemäß kann die Abspaltung der Schutzgruppe vorzugsweise chemisch oder photoinduziert erfolgen. Als hierfür geeignete Schutzgruppen kommen insbesondere die folgenden Verbindungen Nitropirenoyl, Pyrenylmethoxycarbonyl, Nitroveratryl, Nitrobenzyl, Dimethyldimethoxybenzyl, 5-Brom-7-nitroindolinyl, o-Hydroxy-α-methylcinnamoyl und 2-Oxymethylenanthrachinon in Betracht, wobei diese Aufzählung nicht abschließend ist und keinesfalls eine Beschränkung der vorliegenden Erfindung darstellen soll.

Es ist vorteilhaft, wenn das erfindungsgemäße Verfahren kontinuierlich im Durchfluss betrieben wird. Folglich betrifft eine bevorzugte Ausführungsform ein Verfahren, bei dem die Schritte b) bis d) kontinuierlich im Durchfluss erfolgen. Im Durchflussverfahren können zum einen die NTPs einfach zu- und abgeführt werden, und zum anderen kann der Verbrauch an Enzym und NTPs reduziert werden.

Nach einer weiteren bevorzugten Ausführungsform kann die Sensoroberfläche mit einer kopplungsfähigen Substanz beschichtet werden. Typischerweise werden hierzu die Sensor-Oberflächen, wie z.B. solche aus Siliziumdioxid, in eine Lösung von bifunktionellen Molekülen, die beispielsweise eine Halogensilan- oder Alkoxysilangruppe zur Kopplung an die Trägeroberfläche aufweisen, getaucht, sodass sich eine sich selbst organisierende Monoschicht (SAM) bildet, durch welche die kovalente Bindung zwischen Sensoroberfläche und Rezeptor erzeugt wird. Geeignete bifunktionelle Moleküle für die Kopplung einer Vielzahl von unterschiedlichen Rezeptor-Molekülen an eine Vielzahl von Trägeroberflächen sind dem Fachmann gut bekannt, vgl. z.B. EP 1456417 A2.

Die als Sondenmoleküle erfindungsgemäß vorgesehenen DNA-Moleküle können mittels gängiger Druckgeräte aufgebracht und immobilisiert werden.

Auf derart hergestellten Oberflächen können unter Anwendung etablierter Verfahren Hybridisierungen durchgeführt werden.

Eine zur Durchführung des erfindungsgemäßen Verfahrens geeignete Vorrichtung umfasst an einer festen Phase direkt oder indirekt wie z.B. über eine geeignete Kopplungsmatrix immobilisierte einzelsträngige DNA-Sonden als Primer, die vorzugsweise unter Ausbildung eines Mikroarrays rasterartig angeordnet sind. Unter geeigneten Bedingungen bildet sich an der DNA-Sonde im Falle des Vorhandenseins eines zu der Sondensequenz komplementären Nukleinsäureanalyten in der zu untersuchenden Probe ein Hybridisierungskomplex. Die DNA-Polymerase bzw. das funktionelle Fragment derselben wie z.B. das Klenow-Fragment der DNA-Polymerase I ist nun in der Lage, in Anwesenheit einer der vier Nukleosidtriphosphate (dATP, dCTP, dTTP, dGTP) den einzelsträngigen Bereich aufzufüllen.

Sofern in der zu analysierenden Probe kein zur DNA-Sonde komplementärer Analyt vorhanden ist, kommt es zumindest in diesem Verfahrensschritt an dieser Stelle zu keiner DNA-Extension und folglich auch zu keiner Generierung eines für die Sequenzierung geeigneten Signals. Die Sequenzierung eines RNA-Analyten unter Verwendung einer RNA-abhängigen DNA-Polymerase als einziges Enzym erfolgt analog unter Ausbildung eines RNA/DNA-Hybridisierungskomplexes.

Die Signale bzw. Messdaten werden von mindestens einem Sensor erfasst, wobei der Sensor monolithisch in der festen Phase integriert und ausgewählt ist aus Feldeffekttransistoren, wobei ionensensitive Feldeffekttransistoren (ISFET) besonders bevorzugt sind.

Es ist bevorzugt, dass die erfindungsgemäß geeignete Vorrichtung eine Mikrokompartimentierung aufweist. Dabei sind Kanäle auf dem Detektorchip aufgebracht, sodass auf einem Chip parallel mehrere verschiedene Nukleinsäureanalyten gemessen werden können. Die Kanäle können z.B. Reihen von Sensorelementen versorgen.

Nach einer weiteren bevorzugten Ausführungsform des erfindungsgemäßen Verfahrens wird daher die Durchführung einer Parallelmessung von identischen Arrays ermöglicht, um die Kosten pro Analyse auf diese Weise drastisch senken zu können. Dazu wird der Chip durch Mikrokanäle in bevorzugt identische Kompartimente unterteilt.

Nach einer weiteren bevorzugten Ausführungsform umfasst die erfindungsgemäß geeignete Vorrichtung Mittel zur Erzeugung eines elektrischen Feldes, mit dessen Hilfe die dNTPs zugegeben oder entfernt werden können.

Die Signale des Sensors werden durch eine Aufnahmeeinheit festgehalten. Diese Aufnahmeeinheit besitzt vorteilhafterweise einen Konverter zur Umwandlung analoger Detektorsignale in digitale Werte, die gespeichert werden. Zur Auswertung der digitalen Werte kann ein gewöhnlicher Mikroprozessor verwendet werden.

Die mögliche Auswertung der geeigneten Signale im Rahmen des erfindungsgemäßen Verfahrens über einen handelsüblichen Computer weist den weiteren Vorteil auf, dass über geeignete Programme eine weitgehende Automatisierung der Datenauswertung sowie -speicherung möglich ist.

Nach einer bevorzugten Ausführungsform der erfindungsgemäß geeigneten Vorrichtung ist eine Vielzahl unterschiedlicher DNA-Sonden unter Ausbildung eines Mikroarrays rasterartig angeordnet, wobei jedem immobilisierten DNA-Sondentyp bzw. jedem spezifischen Detektionsbereich ein Sensor zugeordnet ist.

Die Trägereinheit der erfindungsgemäß geeigneten Vorrichtung besteht im Wesentlichen aus einem Halbleitermaterial mit einer integrierten, vorzugsweise mehrere Detektoren umfassenden Detektorschicht.

Die Herstellung eines erfindungsgemäß geeigneten Sensors kann unter Anwendung des an sich bekannten CMOS (complementary metaloxide semiconductor)-Verfahrens erfolgen.

Als weitere ebenfalls geeignete Herstellungsverfahren kommen z.B. NMOS-Prozesse oder Bipolar-Prozesse in Betracht (s. z.B. S. Wolf, Silicon Processing for the VLSI ERA Vol.1, Lattice Press, Sunset Beach (1986)). Ferner besteht die insbesondere nach Kostengesichtspunkten interessante Möglichkeit der Herstellung eines erfindungsgemäßen Biosensors auf der Basis von organischen Halbleitern (s. z.B. EP-A-1 085 319).

Wenn man als Trägereinrichtung für die DNA-Sonden und die Ausbildung der Sensoren ein monolithisch integrierbares Halbleitermaterial verwendet, lässt sich auch eine monolithisch integrierte Schaltung auf dem selben Substrat herstellen, wodurch in unmittelbarer Nähe zum Untersuchungsobjekt eine Vorverarbeitung der elektronischen SensorAusgangssignale erfolgen kann.

Auf dem Sensorchip kann man erfindungsgemäß auch Detektoren vorsehen, die nicht mit DNA-Sonden bedruckt oder beschichtet sind. Das dient dazu, um eine Negativkontrolle zu ermöglichen.

In einer besonders bevorzugten Ausführungsform ist vorgesehen, dass sofern keine dNTP eingebaut werden, dies als Referenz- bzw. Kontrollwerte gespeichert werden kann, um dann beim eigentlichen Detektionsereignis die Möglichkeit zu haben, die aufgenommenen "Störsignale" aus dem Detektionssignal herauszurechnen.

Das allgemeine erfindungsgemäße Verfahren ist für verschiedene Anwendungsgebiete geeignet, beispielsweise zur Genomsequenzierung sowie zur Sequenzierung von hochvariablen humanen Genen, insbesondere für forensische Analysen oder Analysen im Bereich der Transplantationsmedizin.

Die vorliegende Erfindung wird nachfolgend anhand von Beispielen näher erläutert.

Die erfindungsgemäß geeignete Vorrichtung ist im Rahmen eines CMOS-Prozesses hergestellt worden. Ein Kratzschutz ist im Bereich des Sensors entweder scharfkantig oder stufenweise heruntergeätzt, so dass die DNA-Sonden in einem vertieften Bereich angeordnet sind. Die nicht der eigentlichen Detektion dienenden Kratzschutz-Oberflächen des Sensorchips können durch Aufbringung von z.B. Edelmetall oder hydrophoben/hydrophilen Materialien modifiziert sein.

### Herstellung eines erfindungsgemäßen Sensorchips

Der Sensor wird unter Verwendung von 6''(Inch) Wafern mit einem 0,5 µm CMOS-Prozeß gefertigt. Es erfolgt eine Auflagerung und Strukturierung einer Siliziumdioxid-Schicht. Anschließend werden die weiteren üblichen CMOS-Schritte durchgeführt, wie z.B. das Aufbringen einer Verdrahtungsschicht und die Oberflächenpassivierung (Kratzschutz).

### Beschichtung des CMOS-Sensors

Der wie oben hergestellte CMOS-Sensor wird durch Tauchen in eine Lösung von 1% GOPS (Glycidoxypropyltriethoxysilan) und 0,1% Triethylamin in Toluol für eine Zeitdauer von ca. 2 Stunden mit dem Silan beschichtet. Anschließend wird der Chip aus der Lösung entnommen und nach kurzem Abtropfen bei 120° C für eine Zeitdauer von etwa 2 Stunden im Trockenschrank fixiert. Gewünschtenfalls kann der so beschichtete Chip bis zur Biokonjugation unter Feuchtigkeitsausschluß gelagert werden.

### Biokonjugation mit DNA-Sonden

Unter Anwendung herkömmlicher Techniken der oben beschriebene Sensor mit DNA-Sonden bedruckt. Die DNA-Sonden werden hierfür in einer Konzentration von 5 µM in PBS-Puffer gelöst bereitgestellt.

Nach dem Bedrucken wird die Kopplungsreaktion bei 50°C in einer feuchten Kammer fortgesetzt. Anschließend werden die Sensoren mit destilliertem Wasser gespült und sodann zum Trocknen mit Methanol gewaschen. Etwaige verbleibende Lösungsmittelreste werden abschließend durch Verdunsten unter dem Abzug entfernt.

### Hybridisierung

Der Reaktionsansatz wird in einem Puffer (5 x SSPE, 0,1% SDS, 12 µl) für eine Zeitdauer von 2 Stunden bei 50° C in einer Reaktionskammer auf dem Chip hybridisiert. Anschließend wird mit 2 x SSPE 0,1% SDS gespült und der Chip durch Waschen in Wasser gereinigt.

### Extension

Dem Reaktionsansatz werden 10 mmol dATP in Standard-PCR-Puffer sowie 0,3 µl Taq-Polymerase (Quiagen, Hilden, Deutschland, 5 U/µl) zugegeben. Anschließend wird der Ansatz bei 72°C inkubiert. Der Einbau eines jeden dATPs führt zur Freisetzung von Pyrophosphatanionen, deren Ansäuerung des umgebenden Milieus lokal am Ort der Enzymreaktion mit dem integrierten ISFET-Sensor detektiert wird. Weitere Extensionsrunden mit den anderen dNTPs werden durchgeführt. Anschließend wird diese Abfolge von Verfahrensschritten mehrfach wiederholt, bis im Wesentlichen keine neuen Messdaten mehr generiert werden.

### Sequenzierung

Die Bestimmung der Reihenfolge der eingebauten NTPs und damit der Sequenz des zu bestimmenden DNA-Analyten erfolgt durch zeit- und ortsspezifische Zusammenstellung und Auswertung der zuvor erhaltenen Sensordaten.

## Patentansprüche

1. Verfahren zur markierungsfreien Sequenzierung eines Nukleinsäure-Einzelstranges, bei dem lediglich eine DNA-Polymerase oder ein funktionelles Fragment derselben eingesetzt wird, umfassend die folgenden Schritte:
a) Inkubation von auf einer festen Phase immobilisierten DNA-Sonden mit einzelsträngigen Nukleinsäureanalyten zur Ausbildung eines Hybridisierungskomplexes;
b) Zugabe eines der vier Nukleosidtriphosphate dATP, dCTP, dGTP und dTTP;
c) Einbau der Nukleosidtriphospate in Anwesenheit der DNA-Polymerase oder des Fragments derselben, wodurch Pyrophosphatanionen freigesetzt und von einem Sensor detektiert werden;
d) Entfernung überschüssiger Nukleosidtriphosphate;
e) Wiederholung der Schritte b) bis d) unter Zugabe jeweils anderer Nukleosidtriphosphate; sowie optional
f) Mehrfache Wiederholung der Schritte b) bis e); und
g) Bestimmung der Sequenz des Nukleinsäureanalyten auf der Basis der Messdaten, die im jeweiligen Schritt c) erfasst worden sind,
wobei die Detektion in Schritt c) durch mindestens einen Sensor erfolgt, der monolithisch in der festen Phase integriert ist.

2. Verfahren nach Anspruch 1, bei dem der mindestens eine Sensor ein ionensensitiver Feldeffekttransistor (ISFET) ist.

3. Verfahren nach Anspruch 1 oder 2, bei dem die Schritte b) bis d) kontinuierlich im Durchfluss erfolgen.

4. Verfahren nach einem der vorhergehenden Ansprüche, bei dem die eingesetzten Nukleosidtriphosphate eine abspaltbare 3'OH Schutzgruppe aufweisen, deren Abspaltung jeweils vor dem nächsten Verfahrensschritt b) erfolgt.

5. Verfahren nach Anspruch 4, bei dem die 3'OH Schutzgruppen der Nukleoside chemisch oder photoinduziert abgespalten werden.

6. Verfahren nach einem der vorhergehenden Ansprüche, bei dem die Verfahrensschritte b) und/oder d) unter Nutzung eines elektrischen Feldes erfolgen.
